# EUROPEAN PATENT APPLICATION

(11) **EP 1 258 256 A1**
(43) Date of publication of application: **20.11.2002**
(21) Application number: 02253428.3
(22) Date of filing: 16.05.2002
(51) Int. Cl.: A61L 15/44, A61L 15/58

(54) **Adhesive bandage**

(30) Priority: 17.05.2001 BR 0102637
(71) Applicant: JOHNSON & JOHNSON INDUSTRIA E COMERCIO LTDA., Butanta 05501 Sao Paulo, SP (BR)
(72) Inventor: Lafratta, Keli Cristina, Sao Jose dos Campos-SP (BR); Rangel, Fabio Eduardo Franca, Jardim Leonidia Jacarei-SP (BR); Aledo, Maria Aparecida de Carvalho Scamilla, Sao Jose dos Campos-SP (BR)
(74) Representative: Mercer, Christopher Paul

(57) **Abstract**

Adhesive bandage, comprising an adhesive tape (10, 60) having an external surface and an internal surface, the latter presenting two adhesive end portions (13a) to be seated on and attached to the user's skin, and a median portion (13b) that will be seated on the skin area to be treated, said median portion (13b) carrying a plurality of homogeneously distributed microcapsules (30), which will contact the skin area to be treated, and which are formed in a pharmaceutically acceptable material, said microcapsules (30) being ruptured by external pressure or by dissolution in liquids exuded from the skin, and each microcapsule (30) containing at least one active agent presenting at least one of the anesthetic, antiseptic, hemostatic, healing, and antibiotic functions.

## Description

### Field of the Invention

The present invention refers to an adhesive bandage, to be applied to wounds of the human skin and comprising an adhesive tape to which is optionally attached an absorbent substrate, in the form of a pad for contacting the skin in the injured region, said adhesive tape carrying at least one active agent with antiseptic, hemostatic, analgesic, healing, therapeutic, and other properties, which will be released when contacting the user's skin upon application of the bandage.

### Background of the Invention

There are known in the art the adhesive bandages of the type mentioned above and having the pad coated with a wax layer, generally based on semi-solid hydrocarbons and which defines a vehicle carrying an active agent with antiseptic or healing properties, to be applied to the skin region that will be covered by the pad of the bandage.

Although allowing the bandage to exert at least one additional function besides that of protecting the wound, this solution that employs a wax layer as a vehicle for storing and dispensing the active agent presents the disadvantages of eliminating, at least partially, the capacity of the pad to exert its function of absorbing the liquids exuded from the skin, and of requiring an special package to prevent the wax from migrating to the wrap, which considerably increases the cost of the bandage.

Other known bandage of the type considered herein is described in US Patent No. 4,117.841 and comprises one or more capsules, which enclose an active agent to be applied to the skin and which are disposed on the pad of the adhesive bandage, and being there maintained by respective blisters formed in a detachable retaining strip, which is affixed to the adhesive strip to cover the pad. In this constructive solution, just before the application of the bandage, the user presses the blisters with his fingers, causing the rupture of the capsules and the flow of the active agent to the pad. Subsequently, the retaining strip is detached so that the pad containing a new load of active agent may be applied to the region to be protected and treated.

Moreover, in the above solution, it is necessary the provision of a relatively complex and expensive construction, which requires one blister for each capsule or load of active agent. However, in the case in which each blister contains a load of the non-encapsulated active agent, said load is insulated from the pad by an insulating film, which is torn upon pressing the respective blister. Besides the constructive complexity, this known solution leads to the provision of relatively large loads of active agent, each load being retained in a respective blister. This arrangement makes impracticable to obtain a homogeneous distribution of the active agent along the whole surface of the pad, which needs a minimal quantity of the active agent to promote the required superficial dispersion. Thus, a certain not-negligible quantity of the active agent remains embedded in the pad, having no function in relation to the injured area. The active agent is firstly transferred to the structure of the pad and only then it will partially contact the skin.

Besides leading to an expensive loss of active agent, the solution of US Patent No. 4,899,739 considerably limits the versatility of providing the capsules with different active agents, usually mutually incompatible to be contained in the same capsule or blister.

Other known solution is described in US Patent 4,117,841. In this case, a load of active agent is positioned between the bandage and a strip of impermeable material, which is affixed to the adhesive tape and which will be manually pressed by a plurality of sharp-pointed elements disposed against the adhesive tape and thus capable of promoting rupture of the impermeable strip, allowing the active agent, either in the liquid or gel form, to flow and contact the skin when the bandage is applied. This construction requires the provision of both the impermeable strip and the sharp-pointed elements, making difficult and expensive to produce the bandage. This solution is further deficient as to the versatility in providing different active agents.

### Objectives of the Invention

It is a generic object of the present invention to provide an adhesive bandage of the type considered herein, which has a relatively simple construction and low manufacturing cost, and which presents an efficient and adequate distribution of the active agent in the pad, and also the possibility of providing different active agents along the area designed to cover the injured region.

It is a further object of the present invention to provide an adhesive bandage, such as defined above, which may be applied, releasing the active agent to the region to be treated, without requiring the user to remove any blocking element of the active agent.

It is still a further object of the invention to provide an adhesive bandage with the above characteristics, which allows releasing the active agent to the injured region, by simply applying the bandage to the user's skin.

### Summary of the Invention

The above objectives are achieved by providing an adhesive bandage of the type comprising an adhesive tape, having an external surface and an internal surface, the latter presenting two adhesive end portions to be seated on and attached to the user's skin, and a median portion, to be seated on the skin area to be treated.

According to the invention, the median portion carries a plurality of homogeneously distributed microcapsules, which will contact the skin area to be treated and which are formed in a pharmaceutically acceptable material, said microcapsules being ruptured by external pressure or by dissolution in liquids exuded from the skin, and each microcapsule containing at least one active agent presenting at least one of the anesthetic, antiseptic, hemostatic, healing and antibiotic function.

The technical solution defined above allows obtaining an adhesive bandage with a conventional basic construction, but containing, in its structure, elements that may be selectively ruptured, in order to homogeneously and efficiently release to the wound a load of an active agent with therapeutic, antiseptic or analgesic functions.

### Brief Description of the Drawings

The invention will be described below, with references to the enclosed drawings, in which:
Figure 1 is a perspective view of an adhesive bandage constructed according to a first embodiment of the invention;
Figure 2 illustrates an enlarged and exploded detail of the median portion of the adhesive bandage illustrated in figure 1;
Figure 3 is a schematic cross-sectional view of the median portion of the bandage of figures 1 and 2;
Figure 4 is an enlarged and exploded perspective view of the median portion of an adhesive bandage constructed according to a second embodiment of the invention;
Figure 5 is a schematic cross-sectional view of the median portion of the bandage of figure 4;
Figure 6 is a schematic cross-sectional view of the median portion of an adhesive bandage constructed according to a third embodiment of the invention;
Figure 7 is a schematic cross-section view of the median portion of an adhesive bandage constructed according to a fourth embodiment of the invention; and
Figure 8 is a schematic cross-sectional view of the median portion of an adhesive bandage constructed according to a fifth embodiment of the invention.

### Description of the Illustrated Embodiments

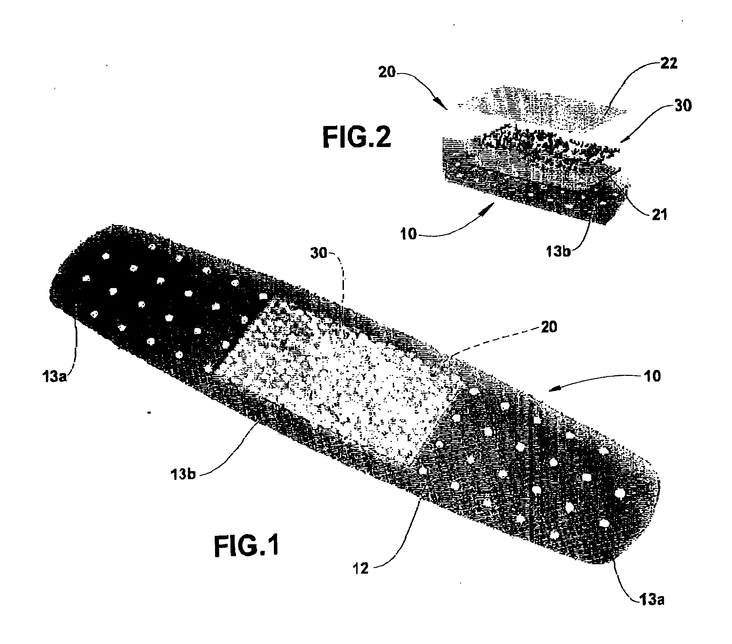

According to the invention, the adhesive bandage is of the type comprising an adhesive tape 10 which in the embodiments of figures 1-7 is formed in a manner well known in the art. The adhesive tape 10 presents an external surface 11 and an internal surface 12, the latter being coated with an adhesive file 13 and having two adhesive end portions 13a, to be seated and affixed to the user's skin, and a median portion 13b, to be seated on the skin area to be treated.

According to the embodiments illustrated in figures 1-6, the median portion 13b of the internal surface of the adhesive tape 10 carries a pad 20 usually formed by one or more sheets 21 of a non-woven material, which sheets 21 are externally coated with a non-adhesive perforated film, which is peripherally bonded to the lower surface of the adhesive tape 10 usually constructed in a polymeric material, such as polyethylene (Delnet™), or the like, so as to prevent the pad 20 from adhering to the wound when applying the bandage.

In the specific embodiment shown in figures 1, 2 and 3, between the innermost sheet 21 of the pad 20 and the non-adhesive perforated film 22, there is provided a plurality of microcapsules 30, which are homogeneously distributed along the whole innermost contact surface of the sheet 21 of the bandage. In this specific embodiment of figures 1, 2 and 3, the non-adhesive perforated film may even be eliminated, when using a microcapsule containing a material that will form a non-adhesive barrier after rupture, or when part of the microcapsules has sufficient resistance against rupture and therefore may act as a non-adhesive barrier between the wound and the pad. The microcapsules 30 are made from a pharmaceutically acceptable non-toxic material, preferably selected from gelatin, alginates and chitosan.

In a particular and exemplary way, the microcapsules 30 may be constructed, besides gelatin, in gum Arabic, carrageen gum, dextrin, ethyl cellulose, modified edible amide, guar gum, hydroxypropylcellulose, hydroxypropylmethylcellulose, methylcellulose, methylethylcellulose, microcrystalline wax, polyethylene glycol, polyvinyl acetate, polyvinylpyrrolidone, sodium alginate, potassium alginate, povidone, sodium carboxymethylcellulose, synthetic wax and natural terpene resin.

The microcapsules 30 are preferably constructed with an average dimension ranging from about 0.010 mm to about 1.000 mm, presenting a wall thickness that is shaped and dimensioned to allow its rupture, upon being selectively and externally pressed by the user, before or after applying the bandage to the skin, or said microcapsules may be further constructed in order to be progressively dissolved by the liquids exuded from the skin, in the wound region, so as to allow releasing to said wound region a load of active agent contained inside said microcapsules 30 and presenting at least one of the anesthetic, antiseptic, hemostatic, healing and antibiotic solutions.

Possible active agents that may be used inside the microcapsules 30 are: benzocaine, lidocaine, pramoxine, benzethonium chloride, benzalkonium chloride, chlorhexidine, iodopolividone, calendula, eucalyptus, aluminum sulphate, ferrous sulphate, alginates, allantoin, neomycin, bacitracin, sulfa, calamine, irgasan, camphor, menthol; it should be understood that these active agents may be presented in any of the forms defined by: solution, dispersion, gel, ointment, paste and/or powder.

In the constructive arrangement illustrated in figures 1, 2 and 3, the use of the bandage generally requires the actuation of the user, before or during the application, for pressing the microcapsules 30, in order to promote the rupture thereof and consequently the flow of the active agent inside the pad, allowing it to migrate towards the non-adhesive perforated film 22, and thence to the user's skin in the injured region. As a function of the microcapsules 30 being homogeneously distributed along the area of the pad 20, the rupture of said microcapsules produces a flow of active agent in the whole area of the pad, not requiring an internal transversal migration of the active agent, so that it may achieve the whole superficial contact area of the non-adhesive perforated film 22 and act on the wound covered with the bandage.

It should be understood that the microcapsules 30 may be constructed, as already mentioned, to dissolve when submitted to humidity by the liquids exuded from the wound region, in which situation the microcapsules may be designed and dimensioned to contain different active agents that will be progressively released in different phases of the wound treatment, as a function of the degree and time of humidity to which they are submitted.

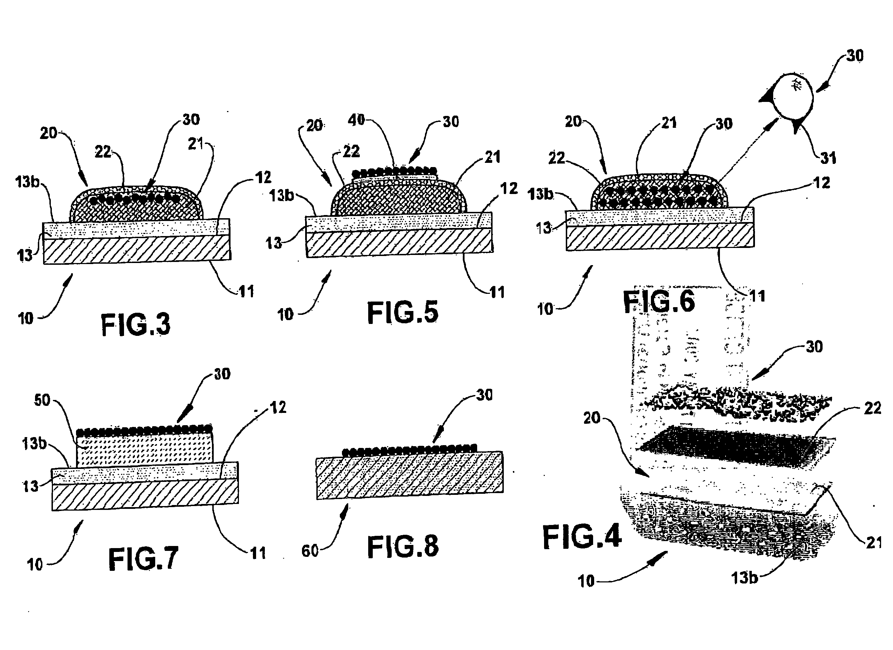

In the embodiment illustrated in figures 4 and 5, the microcapsules 30 are seated on and attached to a non-adhesive perforated film 22, by means of an adhesive layer 40, which is applied to the external contact surface of said non-adhesive perforated film 22, as better illustrated in figure 5.

The adhesive layer 40 may be obtained from a polymeric material, preferably acrylic, or it may be formed in a viscous gel film, preferably polyvinylpirrolidone or polyvinyl alcohol.

In this second embodiment of the invention, illustrated in figures 4 and 5, the microcapsules 30 are disposed in order to directly contact the skin in the wound region, upon applying the bandage, or they may be previously ruptured by pressure exerted by the user, or they are dissolved by the liquids exuded from the wound, as previously mentioned.

In a third constructive embodiment illustrated in figure 6, the microcapsules 30 are disposed in the interstices of the sheet 21 by using a polymeric binder 31, preferably acrylic. Also in this case, the microcapsules 30 may be formed by aggregating sharp external radial projections 31, which are obtained by an adequate stiffened material, and which, when the pad is manually pressed before or during the application of the bandage, perforate the adjacent microcapsules, producing the flow of active agent inside the pad and towards the wound, upon applying the bandage to the user's skin.

In the embodiment illustrated in figure 7, the median portion 13b of the internal surface of the adhesive tape 10 carries a pad 50 consisting of hydrogel in which case the microcapsules 30 are directly adhered to the surface of the pad 50 to be seated on the wound. In this embodiment, the adhesion of the microcapsules 30 to the pad 50 is obtained by imparting a slightly adhesive characteristic to the hydrogel composition.

Still another possible embodiment of the present adhesive bandage is illustrated in figure 8, in which the microcapsules 30 are directly adhered to the median portion of the internal surface of an adhesive tape 60, consisting of hydrocolloid.

## Claims

1. An adhesive bandage, comprising an adhesive tape (10, 60), which has an external surface and an internal surface, the latter presenting two adhesive end portions (13a) to be seated on and attached to the user's skin, and a median portion (13b) to be seated on the skin area to be treated, **characterized in that** the median portion (13b) carries a plurality of homogeneously distributed microcapsules (30), which will contact the skin area to be treated, and which are formed in a pharmaceutically acceptable material, said microcapsules (30) being ruptured by external pressure or by dissolution in liquids exuded from the skin, each microcapsule (30) containing at least one active agent presenting at least one of the anesthetic, antiseptic, hemostatic, healing, and antibiotic functions.

2. Adhesive bandage, according to claim 1, **characterized in that** the microcapsules 30 are made of non-toxic materials selected from gelatin, alginates, and chitosan.

3. Adhesive bandage, according to claim 2, **characterized in that** the microcapsules (30) are obtained from the group consisting of one of the following materials, besides gelatin: gum Arabic, carrageen gum, dextrin, ethylcellulose, modified edible amide, guar gum, hydroxypropylcellulose, hydroxypropyl methylcellulose, methylcellulose, methylethylcellulose, microcrystalline wax, polyethylene glycol, polyvinyl acetate, polyvinylpirrolidone, sodium alginate, potassium alginate, povidone, sodium carboxymethylcellulose, synthetic wax, and natural terpene resin.

4. Adhesive bandage, according to claim 3, **characterized in that** the microcapsules (30) present an average dimension from about 0,010 mm to about 1,00 mm.

5. Adhesive bandage, according to claim 1, **characterized in that** the active agent is selected from the group consisting of benzocaine, lidocaine, pramoxine, benzethonium chloride, benzalkonium chloride, chlorhexidine, iodopolividone, calendula, eucalyptus, aluminum sulphate, ferrous sulphate, alginates, allantoin, neomycin, bacitracin, sulfa, calamine, irgasan, camphor, menthol.

6. Adhesive bandage, according to claim 5, **characterized in that** the active agent is presented in any of the forms defined by solution, dispersion, gel, ointment, paste, and powder.

7. Adhesive bandage, according to claim 1, presenting the median portion (13b) of the internal surface thereof provided with a pad (20) formed by multiple non-woven sheets (21), **characterized in that** the microcapsules (30) are disposed between at least two sheets (21) of the pad (20).

8. Adhesive bandage, according to claim 1, and presenting the median portion (13b) of the internal surface thereof provided with a pad (20) formed by at least one non-woven sheet (21), with its surface to be seated on the wound being coated with a non-adhesive perforated film (22), **characterized in that** the microcapsules (30) are disposed between the innermost sheet (21) of the bandage and the non-adhesive perforated film (22), or between the interstices of the sheet (21), or also between two or more layers of said sheet (21).

9. Adhesive bandage, according to claim 1, and presenting the median portion (13b) of the internal surface thereof provided with a pad (20) formed by at least one non-woven sheet (21), with its surface to be seated on the wound being coated with a non-adhesive perforated film (22) **characterized in that** the microcapsules (30) are seated on and attached to the non-adhesive perforated film (22) by means of an adhesive layer (40) applied thereto.

10. Adhesive bandage, according to claim 9, **characterized in that** the adhesive layer (40) is made of a polymeric material, preferably acrylic.

11. Adhesive bandage, according to claim 9, **characterized in that** the adhesive layer (40) is obtained in a viscous gel film, preferably polyvinylpyrrolidone or polyvinyl alcohol.

12. Adhesive bandage, according to claim 1, and presenting the median portion (13b) of the internal surface thereof provided with a pad (50) consisting of hydrogel, **characterized in that** the microcapsules (30) are directly adhered to the surface of the pad (50) to be seated on the wound.

13. Adhesive bandage, according to claim 12, **characterized in that** the adhesion of the microcapsules (30) to the pad (50) is obtained by the slightly adhesive composition of the hydrogel.

14. Adhesive bandage, according to claim 1, **characterized in that** the microcapsules (30) are directly adhered to the median portion of the internal surface of the adhesive bandage (60).

15. Adhesive bandage, according to claim 14, **characterized in that** the adhesive tape (60) consists of hydrocolloid.
